# FASCICULE DE BREVET EUROPEEN

(11) **EP 4 125 962 B1**
(45) Date de publication et mention de la délivrance du brevet: **19.02.2025**
(21) Numéro de dépôt: 21714215.7
(22) Date de dépôt: 29.03.2021
(51) Int. Cl.: A61K 35/36, A61K 35/33, A61L 27/24, A61P 17/02, A61K 8/98, A61L 24/00

(54) **BIOMATÉRIAU COMPRENANT UNE MATRICE POREUSE RÉSORBABLE ET PROCÉDÉ DE FABRICATION ASSOCIÉ**
BIOMATERIAL MIT EINER PORÖSEN RESORBIERBAREN MATRIX UND ZUGEHÖRIGES HERSTELLUNGSVERFAHREN
BIOMATERIAL COMPRISING A POROUS RESORBABLE MATRIX AND ASSOCIATED MANUFACTURING METHOD

(30) Priorité: 30.03.2020 FR 2003125
(43) Date de publication de la demande: 08.02.2023
(73) Titulaire: Université Grenoble Alpes, 38400 Saint-Martin-d'Hères (FR); Commissariat à l'Energie Atomique et aux Energies Alternatives, 75015 Paris (FR); Centre national de la recherche scientifique, 75016 Paris (FR)
(72) Inventeur: JOBEILI, Lara Marwa, 38000 GRENOBLE (FR); LELLOUCH, Alexandre Gaston Mickaël, 92300 LEVALLOIS-PERRET (FR); RACHIDI, Walid, 38240 MEYLAN (FR); LANTIERI, Laurent Alexandre, 75007 PARIS (FR)
(74) Mandataire: Hautier IP
(86) Numéro de dépôt international: PCT/EP2021/058151
(87) Numéro de publication internationale: WO 2021/198177

(56) Documents cités:
- FR-A1- 2 809 313
- FR-B1- 2 809 313
- US-A- 5 282 859
- US-A- 5 945 101
- US-A- 6 153 292
- US-A1- 2006 135 921
- VAISSIERE G ET AL: "COMPARATIVE ANALYSIS OF DIFFERENT COLLAGEN-BASED BIOMATERIALS AS SCAFFOLDS FOR LONG-TERM CULTURE OF HUMAN FIBROBLASTS", MEDICAL AND BIOLOGICAL ENGINEERING AND COMPUTING, SPRINGER, HEILDELBERG, DE, vol. 38, no. 2, 1 March 2000 (2000-03-01), pages 205 - 210, XP000914401, ISSN: 0140-0118, DOI: 10.1007/BF02344778

## Description

### DOMAINE TECHNIQUE

La présente invention concerne le domaine des biomatériaux comprenant du collagène et leur fabrication. Elle trouve pour application particulièrement avantageuse le domaine des substituts dermo-épidermiques.

### ETAT DE LA TECHNIQUE

Les plaies chroniques représentent un problème majeur de santé publique affectant plus de 2 millions de personnes uniquement en France métropolitaine. Ceci inclut les brûlures physiques, par exemple induites par un rayonnement ultra-violet, et chimiques, par exemple induites par des détergents ou des acides, les ulcères, et notamment les ulcères de pression, les ulcères veineux et des ulcères diabétiques. Ces plaies chroniques nécessitent généralement le remplacement des tissus lésés, par exemple par une greffe cutanée.

Pour le cas particulier des greffes de peau, il existe différents types de greffes classés en fonction de l'épaisseur de peau prélevée sur la personne :
- la greffe de peau mince est prélevée par un dermatome électrique avec une épaisseur de 0,2 à 0,3 mm sur une zone donneuse du corps du patient, généralement une face interne de cuisse ou du scalp. Ce prélèvement est ensuite transféré sur une zone receveuse (à l'aide de suture ou d'agrafe) du patient pour une greffe autologue. Cette technique peut être complétée avec l'ajout d'un substitut dermique artificiel tel que l'integra^{®}. Le principal inconvénient réside dans les séquelles induites par le prélèvement et la surface limitée des zones donneuses;
- la greffe de peau totale, ne nécessite pas de prélèvement au dermatome comme pour une greffe de peau mince. Une incision fusiforme est réalisée dans un pli de peau, par exemple le pli sus claviculaire. La peau est ensuite dégraissée (pour permettre une meilleure prise de greffe), puis suturée sur la zone receveuse. Cette technique est considérée comme particulièrement adaptée pour le visage.

Les prélèvements des tissus cutanés dans le cadre des greffes restent toutefois limités, notamment en termes de surface disponible de zones donneuses et des douleurs induites chez la personne prélevée.

Par ailleurs, depuis 2013, les tests de l'efficacité et de la sécurité des produits dermo-cométiques sont interdits sur les animaux. Pour tester ces substances, l'industrie pharmaceutique ou cosmétique doit donc avoir recours à des substituts tissulaires mimant au mieux les propriétés et les réactions d'un tissu natif.

Il existe donc un besoin pour développer un substitut tissulaire au plus proche d'un tissu natif, par exemple pour le traitement de plaies chroniques ou pour des applications pharmacologiques ou cosmétiques.

Il est connu, du document WO 88/10123 A1, un biomatériau comprenant un composé constitué de collagène, de chitosan acétylé et de glycosaminoglycanes. Ce biomatériau peut constituer la couche dermique d'une peau artificielle, et être associé à un pseudo épiderme biodégradable, par exemple un film de chitosan. Toutefois, ce biomatériau reste perfectible pour mimer une peau native.

US 5 945 101 A décrit un matériau comprenant une éponge de collagène réticulé dans lequel des fibroblastes et des kératinocytes ont été ensemencés ainsi que le procédé de fabrication d'un modèle de peau comprenant une matrice de collagène comprenant des fibroblastes et des couches stratifiées de cellules épidermiques différenciées.

Un objet de la présente invention est donc de préparer un biomatériau se rapprochant davantage d'un tissu natif.

Les autres objets, caractéristiques et avantages de la présente invention apparaîtront à l'examen de la description suivante et des dessins d'accompagnement. Il est entendu que d'autres avantages peuvent être incorporés.

### RESUME

Pour atteindre cet objectif, on prévoit un biomatériau (non revendiqué) comprenant une matrice poreuse résorbable formée d'un matériau comprenant du collagène, et présentant un volume intérieur et une surface extérieure.

Avantageusement, le biomatériau comprend au moins un type de cellules biologiques vivantes d'un tissu, disposées dans le volume intérieur et, en alternative ou en complément, sur la surface de la matrice poreuse.

Ainsi, le biomatériau comprend une matrice lui conférant une structure permettant sa manipulation, et des cellules biologiques d'un tissu ensemencées dans la matrice poreuse. Le biomatériau forme donc un substitut tissulaire se rapprochant d'un tissu natif, notamment en termes de structure biologique présente dans le tissu, et de fonctions physiologiques.

En outre, la matrice étant résorbable, la matrice poreuse peut être dégradée pour faciliter l'intégration d'une partie du biomatériau dans l'organisme, et plus particulièrement d'un tissu formé par les cellules biologiques, par exemple suite à une greffe du biomatériau. Le biomatériau forme donc un substitut tissulaire pouvant être greffé sur un organisme, puis agir directement pour réaliser sa fonction de tissu, sans nécessiter en outre le retrait du biomatériau, et plus particulièrement de la matrice poreuse.

Selon un exemple, l'au moins un type de cellules biologiques vivantes d'un tissu sont disposées dans le volume intérieur de la matrice poreuse.

Un premier aspect de l'invention concerne un procédé de fabrication d'un biomatériau selon la revendication 1 comprenant :
- une fourniture d'une matrice poreuse résorbable formée d'un matériau comprenant du collagène, puis
- un ensemencement de la matrice poreuse par au moins un type de cellules biologiques vivantes d'un tissu, puis
- une croissance cellulaire de l'au moins un type de cellules biologiques.

Le procédé permet ainsi d'obtenir le biomatériau selon le premier aspect de l'invention. Par ensemencement et croissance, on peut en outre former un biomatériau de taille et de forme variable en fonction des dimensions de la matrice poreuse. On obtient ainsi un biomatériau formant un substitut tissulaire se rapprochant d'un tissu natif.

Un deuxième aspect concerne l'utilisation d'un biomatériau fabriqué selon le procédé des revendications 1-8, le biomatériau comprenant :
- une matrice poreuse résorbable formée d'un matériau comprenant du collagène, et présentant un volume intérieur et une surface, et
- au moins un type de cellules biologiques vivantes d'un tissu, disposées dans le volume intérieur et, en alternative ou en complément, sur la surface de la matrice poreuse, pour une application in vitro d'au moins une substance choisie parmi une substance pharmaceutique et une substance cosmétique.

### BREVE DESCRIPTION DES FIGURES

Les buts, objets, ainsi que les caractéristiques et avantages de l'invention ressortiront mieux de la description détaillée d'un mode de réalisation de cette dernière qui est illustré par les dessins d'accompagnement suivants dans lesquels :
La figure 1 représente une vue en coupe transversale de la matrice poreuse selon un mode de réalisation de l'invention.
La figure 2 représente un premier ensemencement de la matrice poreuse illustrée en figure 1 par un premier type de cellules biologiques, selon un mode de réalisation de l'invention.
La figure 3 représente la croissance cellulaire des cellules biologiques suite à l'ensemencement illustré en figure 2, selon un mode de réalisation de l'invention.
La figure 4 représente un deuxième ensemencement de la matrice poreuse par un deuxième type de cellules biologiques et une première sous-croissance cellulaire subséquente du deuxième type de cellules biologiques, suite à la croissance cellulaire illustrée en figure 3, selon un mode de réalisation de l'invention.
La figure 5A représente une maturation du deuxième type de cellules biologiques suite à la première sous-croissance cellulaire illustrée en figure 4, selon un mode de réalisation de l'invention.
La figure 5B représente le biomatériau, selon un mode de réalisation de l'invention.
La figure 6 représente les étapes du procédé de fabrication du biomatériau, selon un mode de réalisation de l'invention.
La figure 7 représente une chronologie des étapes du procédé de fabrication du biomatériau, selon un mode de réalisation de l'invention.
La figure 8 représente une image immunohistologique de microscopie à fluorescence en coupe transversale du biomatériau, dans laquelle les noyaux des cellules biologiques et la cytokératine 14 sont marqués.
La figure 9 représente une image immunohistologique de microscopie à fluorescence en coupe transversale du biomatériau, dans laquelle les noyaux des cellules biologiques, la filaggrine et le collagène de type 1 sont marqués.
La figure 10 représente une image immunohistologique de microscopie à fluorescence en coupe transversale du biomatériau, dans laquelle les noyaux des cellules biologiques, la fibrilline 1 et l'élastine sont marqués.

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques. En particulier, les dimensions relatives des cellules biologiques entre elles et par rapport à la matrice poreuse ne sont pas représentatives de la réalité.

### DESCRIPTION DÉTAILLÉE

Avant d'entamer une revue détaillée de modes de réalisation de l'invention, sont énoncées ci-après des caractéristiques optionnelles qui peuvent éventuellement être utilisées en association ou alternativement pour chacun des aspects de l'invention :
- l'au moins un premier type de cellules biologiques peut être disposé au moins dans le volume intérieur de la matrice poreuse, voire uniquement dans le volume intérieur de la matrice,
- l'au moins un premier type de cellules biologiques peut être disposé au moins sur une surface extérieure de la matrice poreuse, voire uniquement sur une surface de la matrice, et de préférence sur une face supérieure de la matrice poreuse,
- la matrice poreuse peut être biocompatible,
- le matériau de la matrice poreuse peut comprendre au moins 90%, de préférence au moins 95 %, et plus préférentiellement au moins 99 % en masse de collagène, par rapport à la masse sèche de la matrice poreuse,
- le matériau de la matrice poreuse peut être exempt de chitosan,
- la matrice poreuse est une éponge hémostatique. Ainsi, la matrice poreuse peut être disponible commercialement et à un coût réduit,
- la matrice poreuse peut être un produit suivant les Bonnes Pratiques de Fabrication (BPF). La matrice poreuse peut être utilisable cliniquement chez l'humain ou l'animal. Le biomatériau peut ainsi être utilisable cliniquement chez l'humain ou l'animal,
- les cellules biologiques peuvent comprendre des cellules choisies parmi des cellules de tissu conjonctif, des cellules épithéliales et endothéliales. Selon un exemple, les cellules de tissu conjonctif sont des cellules dermiques appelées fibroblastes et/ou des cellules du tissu adipeux sous cutané appelées cellules souches du tissu adipeux. Selon un exemple, les cellules épithéliales sont des cellules d'épiderme comprenant les kératinocytes et/ou les mélanocytes. Selon un exemple, les cellules endothéliales sont des cellules des vaisseaux. De préférence, les cellules biologiques sont des cellules humaines ou animales,
- l'au moins un type de cellules biologiques peut être disposé au moins dans le volume intérieur de la matrice poreuse, voire uniquement dans le volume intérieur de la matrice, le biomatériau peut comprendre en outre une matrice extracellulaire au moins dans ledit volume intérieur, la matrice extracellulaire étant sécrétée par l'au moins un premier type de cellules biologiques. Les cellules biologiques et leur matrice extracellulaire permettent de former un tissu dans la matrice poreuse, et se rapprocher encore des propriétés d'un tissu natif,
- un premier type de cellules biologiques peut être disposé dans le volume intérieur de la matrice poreuse, et le biomatériau peut comprendre au moins un deuxième type de cellules biologiques disposé sur la surface, par exemple la face supérieure, de la matrice poreuse, le deuxième type de cellules biologiques pouvant en outre être distinct du premier type de cellules biologiques. Ainsi, le biomatériau peut en outre comprendre une couche cellulaire en surface de la matrice poreuse formant une limite externe d'un tissu, et se rapprocher encore d'un tissu natif. Selon un exemple, le deuxième type de cellules biologiques est un type de cellules épithéliales. Ainsi, le biomatériau présente des propriétés barrières. Le biomatériau permet donc le remplacement de la fonction de barrière, par exemple sur une plaie ne cicatrisant pas spontanément, telle qu'une plaie chronique,
- le premier type de cellules biologiques peut comprendre des cellules dermiques, par exemple des fibroblastes, et le deuxième type de cellules biologiques peut comprendre des cellules épithéliales, par exemple des kératinocytes, voire en outre des mélanocytes. Selon un exemple, le premier type de cellules biologiques peut comprendre en outre au moins un type de cellules biologiques parmi des cellules endothéliales, des cellules immunitaires et des cellules souches du tissu adipeux. Selon un exemple, l'au moins un deuxième type de cellules biologiques peut comprendre en outre des mélanocytes,
- les cellules biologiques sont choisies parmi des cellules dérivées de cultures de lignée cellulaire standard et préférentiellement des cellules primaires isolées d'un échantillon biologique d'un organisme. Selon un exemple, le biomatériau peut comprendre des cellules autologues ou allogéniques. Selon un exemple, les cellules biologiques sont isolées depuis un échantillon de peau d'une personne.

Des caractéristiques du procédé selon l'invention sont énoncées ci-aprés:
- le procédé comprend une hydratation de la matrice poreuse de façon à équilibrer le pH et l'osmolarité de la matrice poreuse, préalablement à l'ensemencement. Selon un exemple, l'hydratation est réalisée avec au moins une solution tamponnée, puis avec un milieu de culture,
- l'hydratation de la matrice poreuse peut être réalisée par une solution d'hydratation présentant un pH compris entre 6,8 et 7,5, et/ou une osmolarité comprise entre 270 et 360 mmol/kg,
- le procédé comprend en outre un séchage de la matrice poreuse entre l'hydratation de la matrice poreuse et l'ensemencement de la matrice poreuse. Selon un exemple, le séchage de la matrice poreuse est effectué par aspiration, par exemple de la solution d'hydratation,
- l'ensemencement de la matrice poreuse peut être réalisé par dépôt d'une suspension de l'au moins un type de cellules biologiques sur la matrice poreuse. Selon un exemple, de l'au moins un type de cellules biologiques peuvent être ensemencés à une densité cellulaire comprise entre 0,5.10⁶ cellules/cm² et 1.10⁶ cellules/cm², les cm² étant donnés en référence à la surface de la matrice poreuse,
- le procédé peut comprendre en outre, après un premier ensemencement par un premier type de cellules biologiques, voire après le premier ensemencement et une partie au moins d'une croissance cellulaire du premier type de cellules biologiques, un deuxième ensemencement de la matrice poreuse par au moins un deuxième type de cellules biologiques, distinct d'un premier type de cellules biologiques ensemencées. Le procédé peut comprendre en outre ensuite une croissance cellulaire de l'au moins un deuxième type de cellule biologiques,
- le procédé peut comprendre en outre un séchage de la matrice poreuse préalablement au deuxième ensemencement de la matrice poreuse,
- le séchage, de préférence chaque séchage, de la matrice poreuse peut comprendre une aspiration de la solution ou du milieu présent dans la matrice poreuse, et le placement de la matrice poreuse sur un support absorbant, de préférence la matrice poreuse est placée sur le support absorbant après l'aspiration,
- le support absorbant peut comprendre une compresse stérile,
- le support absorbant peut comprendre un papier buvard stérile,
- la compresse stérile est de préférence déposée sur un papier buvard stérile,
- le deuxième ensemencement peut être réalisé par dépôt d'une suspension d'au moins un deuxième type de cellules biologiques sur la matrice poreuse,
- les cellules biologiques peuvent comprendre des cellules choisies parmi des cellules de tissu conjonctif et des cellules épithéliales,
- lorsque les cellules biologiques sont des cellules de tissu conjonctif, la croissance cellulaire peut comprendre une immersion de la matrice poreuse dans un milieu de culture. De préférence, la matrice poreuse est entièrement immergée. En outre, la matrice poreuse peut être immergée pendant la majorité de la croissance cellulaire,
- lorsque les cellules biologiques sont des cellules épithéliales, la croissance cellulaire peut comprendre une première sous-croissance pendant laquelle la matrice poreuse est immergée dans un milieu de culture, jusqu'à une confluence des cellules biologiques en surface de la matrice poreuse,
- la croissance cellulaire peut comprendre, suite à la première sous-croissance, une deuxième sous-croissance, pouvant de façon équivalente être désignée comme maturation, durant laquelle la matrice poreuse est placée à une interface entre de l'air et un milieu de culture. La maturation permet la différenciation des cellules épithéliales pour former un épiderme plus complexe, voire complet, et se rapprocher d'un tissu natif

Il est précisé que dans le cadre de la présente invention, le terme « matrice résorbable » désigne le fait que la matrice est biodégradable des cellules, par exemple d'un organisme.

Par une matrice « biocompatible », on entend que la matrice est configurée pour ne pas interférer, voire ne pas dégrader, un milieu biologique au contact duquel la matrice est disposée. Plus particulièrement, la matrice est tolérée par un organisme dans ou sur lequel la matrice est en contact, par exemple par greffe. Plus particulièrement l'organisme présente un faible niveau de réaction inflammatoire, voire une absence de réaction inflammatoire induit par le contact de la matrice, par exemple par greffe.

On entend par un paramètre « sensiblement égal/supérieur/inférieur à » une valeur donnée, que ce paramètre est égal/supérieur/inférieur à la valeur donnée, à plus ou moins 10 % près, voire à plus ou moins 5 % près, de cette valeur.

Par porosité de la matrice, on entend la proportion de vide ou de gaz dans la matrice.

Dans la suite de la description, il sera fait usage de termes tels que, « transversal », « supérieur » et « inférieur ». Ces termes doivent être interprétés de façon relative en relation avec la position normale d'utilisation du biomatériau. Par exemple, dans le cas la matrice poreuse est destinée à être déposée sur un support, la face inférieure correspond à la face destinée à être tournée vers le support. Selon cet exemple, la face supérieure correspond à la face destinée à être tournée à l'opposé du support.

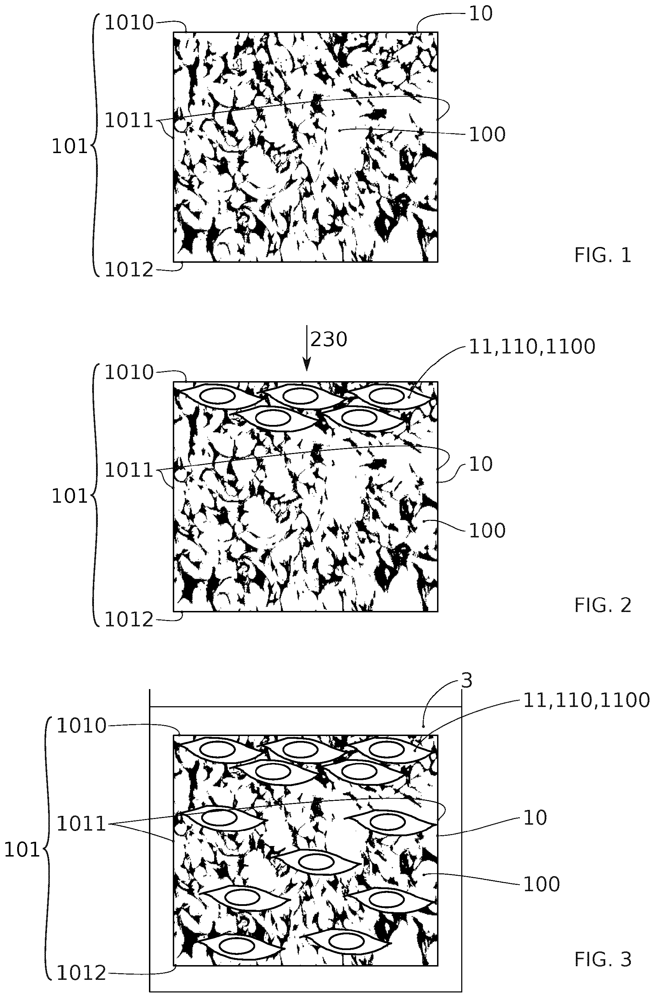

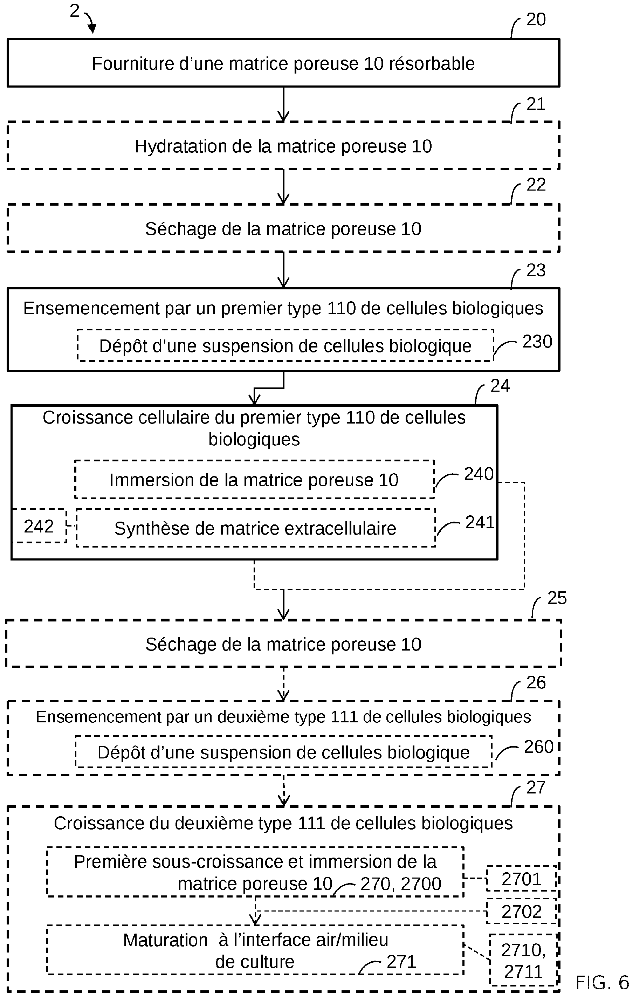

Le procédé 2 de fabrication du biomatériau 1 est maintenant décrit en référence aux figures 1 à 6. À titre d'exemple, le procédé est illustré par la figure 6, où des étapes optionnelles sont indiquées en pointillé.

Le procédé 2 comprend la fourniture 20 d'une matrice poreuse 20. La matrice poreuse 20 présente des pores délimitant un volume intérieur 100, comme par exemple illustré en figure 1. De façon équivalente, le volume intérieur 100 représente le volume de vide ou de gaz à l'intérieur de la matrice 20, et donc la porosité de la matrice. La matrice poreuse 20 présente une surface extérieure 101, comprenant une face supérieure 1010, et une face inférieure 1012. La surface extérieure 100 de la matrice poreuse 10 peut en outre comprendre au moins une face latérale 1011, voire une pluralité de faces latérales 1011, comme par exemple illustré en figure 1.

Selon un exemple, la porosité de la matrice poreuse 10 est homogène, c'est à dire que la porosité par unité de volume est sensiblement identique en en toute portion d'une même taille déterminée de la matrice poreuse 10. Ainsi, on exclut que le volume intérieur soit ménagé dans la matrice poreuse sous la forme d'une cavité. La dimension des pores de la matrice poreuse 10 est de préférence supérieure à la dimension de cellules biologiques 11, et notamment de cellules biologiques eucaryotes. Ainsi, les pores de la matrice poreuse 10 autorisent la migration des cellules biologiques 11 dans le volume intérieur 100. Des caractéristiques additionnelles de la matrice poreuse 10 sont décrites ultérieurement.

La matrice poreuse 10 peut être préparée en vue d'un ensemencement 23 de cellules biologiques 11. Pour cela, le procédé 2 comprend une hydratation 21 configurée pour équilibrer le pH et l'osmolarité de la matrice poreuse autour de valeurs correspondant à l'environnement natif des cellules. Cela permet d'obtenir une osmolarité homogène dans la matrice poreuse 10, et ainsi une meilleure migration et colonisation des cellules 11 ensemencées. Ceci est particulièrement avantageux lorsque la matrice poreuse 10 est épaisse, par exemple lorsque la matrice poreuse 10 est une éponge. À titre d'exemple, pour la fabrication d'un substitut dermo-épidermique, ce pH peut être compris entre 6,8 à 7,5, et l'osmolarité peut être comprise entre de 270 à 360 mmol/kg. Pour comparaison, *in vivo* l'osmolarité cellulaire est sensiblement égale à 290 mmol/kg et le pH cellulaire est sensiblement égal à 7,4. Durant l'hydratation 21, la matrice poreuse 10 peut être immergée dans une solution tamponnée pendant plusieurs heures, voire plusieurs jours. La matrice poreuse 10 peut en outre être immergée dans un milieu de culture 3 configuré pour correspondre à l'environnement natif des cellules. À titre d'exemple, pour la fabrication d'un substitut dermo-épidermique, le milieu de culture 3 peut être un milieu équivalent au milieu du derme.

Suite à son hydratation 21, le procédé 2 comprend un séchage 22 de la matrice poreuse 2 pour favoriser l'adhésion des cellules biologiques 11 sur la matrice poreuse lors de leur ensemencement 23, et donc leur croissance cellulaire 24 subséquente. Par exemple, le séchage de la matrice poreuse 10 peut être effectué par aspiration de la solution d'hydratation.

Le procédé 2 comprend ensuite un premier ensemencement 23 de la matrice poreuse par des cellules biologiques 11 vivantes d'un tissu, et plus particulièrement par au moins premier type 110 de cellules biologiques 11. Ainsi, des cellules biologiques 11 sont ajoutées à la matrice poreuse 10 pour former un biomatériau 11 se rapprochant d'un tissu natif, par rapport à un biomatériau formé uniquement d'un matériau inerte.

Les cellules biologiques 11 peuvent être des cellules eucaryotes, humaines ou animales. Dans la suite, on se réfère à l'exemple non limitatif où les cellules biologiques 11 sont des cellules humaines.

Les cellules biologiques 11 peuvent être choisies parmi des cellules dérivées de cultures de lignée cellulaire standard et des cellules isolées d'un échantillon biologique d'un organisme. Un biomatériau 1 comprenant des cellules 11 dérivées de cultures de lignées cellulaires standard permet de former un substitut tissulaire performant, tout en restant de coût réduit, ce qui est particulièrement avantageux pour des applications pharmaceutiques ou cosmétiques. Les cellules de lignée cellulaire standard peuvent être disponibles sur le marché chez des vendeurs connus de l'homme du métier.

Selon un exemple, les cellules biologiques peuvent être autologues ou allogéniques. Les cellules biologiques allogéniques peuvent par exemple être isolées de résidus opératoire, notamment indépendamment d'une greffe de peau. Un biomatériau 1 comprenant des cellules 11 autologues permet d'être personnalisé pour une personne donnée, ce qui est particulièrement avantageux pour des applications médicales comme par exemple une greffe du biomatériau 1. Dans le cas de cellules autologues, les cellules biologiques peuvent être isolées depuis un échantillon biologique d'une personne, par exemple suite à une ou plusieurs biopsie et notamment une biopsie de peau.

Les cellules biologiques 11 peuvent comprendre des cellules choisies parmi des cellules de tissu conjonctif et des cellules épithéliales. À titre d'exemple, on peut citer comme tissu conjonctif le derme, le tissu adipeux sous cutané, le stroma cornéen, les os, et comme tissu épithélial, l'épiderme et l'épithélium de la cornée. Selon un exemple, les cellules de tissu conjonctif sont des cellules de derme nommées fibroblastes. Selon un exemple, les cellules épithéliales sont des cellules de l'épiderme nommées kératinocytes. Ainsi, et comme décrit plus en détail dans la suite, le biomatériau 1 peut former un substitut dermique, voire dermo-épidermique pour des applications *in vitro* ou *in vivo.*

Dans la suite, on se réfère à l'exemple non-limitatif dans lequel un premier type de cellule comprend, voire est constitué, des cellules de derme, et plus particulièrement des fibroblastes, et un deuxième type de cellule comprend, voire est constitué, des cellules d'épiderme, et plus particulièrement des kératinocytes. Notons que pour la fabrication d'un substitut dermique, voire dermo-épidermique, on peut prévoir que le premier type de cellules biologiques comprenne en addition aux cellules du derme, au moins un type de cellules biologiques parmi :
- des cellules endothéliales, pour obtenir un derme vascularisé,
- des cellules immunitaires, pour obtenir un derme immunocompétent,
- des cellules souches du tissu adipeux, pour obtenir une structure adipeuse sous cutanée.

Selon un exemple, le deuxième type de cellules biologiques peut comprendre en outre des mélanocytes, pour obtenir un épiderme pigmenté. Le biomatériau est ainsi complexifié et se rapproche encore de la peau native, notamment en termes de diversité des cellules biologiques présentes dans le tissu, et de fonctions physiologiques.

Les fibroblastes 1100 peuvent être ensemencés 23 par le dépôt 230 d'une suspension cellulaire, par exemple en goutte à goutte sur la surface 101 de la matrice poreuse, et notamment sur sa face supérieure 1010 selon l'exemple illustré en figure 2. Selon un exemple, les fibroblastes 1100 peuvent être ensemencés à une densité cellulaire comprise entre 500 000 et 1 000 000 cellules/cm², voire sensiblement 800 000 cellules/cm². Notons que selon le type de cellules biologiques ensemencées, la densité cellulaire peut être adaptée. Ainsi, les fibroblastes 1100 peuvent pénétrer dans le volume intérieur 100 de la matrice poreuse 10. Pour faciliter encore l'adhésion des fibroblastes 1100 ensemencées 23, le procédé peut en outre comprendre une étape d'adhésion des cellules 11, durant laquelle la matrice poreuse 10 est laissée à l'air libre pendant un temps déterminé, par exemple plusieurs minutes, voire une heure, voire de préférence dans une atmosphère contenant 5 % en CO₂.

Le procédé 2 comprend ensuite une croissance cellulaire 24 des fibroblastes 1100 ensemencés 23. Les fibroblastes 1100 peuvent se multiplier par division cellulaire au moins dans le volume intérieur 100 de la matrice poreuse 10 pour coloniser au moins en partie le volume intérieur 100, voire sa totalité, comme illustré par la figure 3. Lors de la croissance cellulaire 24, les fibroblastes peuvent en outre synthétiser 241, ou de façon équivalente sécréter, une matrice extracellulaire 112 au moins dans le volume intérieur 100. Les cellules biologiques 11, et notamment les fibroblastes 1100, et leur matrice extracellulaire 112 permettent de former un tissu dans la matrice poreuse 10, et se rapprocher encore des propriétés d'un tissu natif, et notamment d'une peau native. Les fibroblastes 1100 dans le volume intérieur 100 de la matrice poreuse 10, voire les fibroblastes 1100 et leur matrice extracellulaire 112, forment ainsi un substitut dermique équivalent au derme natif.

Pour permettre la croissance cellulaire 24 des fibroblastes 1100, la matrice poreuse 10 avec les fibroblastes 1100 ensemencées 23 peut être immergée au moins partiellement, et de préférence totalement, dans un milieu de culture 3 configuré pour mimer l'environnement natif des fibroblastes 1100, comme illustré en figure 3. La matrice 10 étant poreuse, le milieu de culture 3 est ainsi réparti de façon homogène dans la matrice poreuse 10 pour faciliter la croissance cellulaire 24. Par exemple, le milieu de culture est un milieu équivalent au milieu du derme, c'est à dire dont les propriétés, voire la composition, est sensiblement équivalente au milieu du derme. Durant la croissance cellulaire 24, le milieu de culture 3 peut en outre être renouvelé 242, typiquement à une fréquence de plusieurs fois par semaine. Suite à une première immersion dans un milieu de culture 3 exempt acide ascorbique et de facteur de croissance épidermique (abrégé EGF, de l'anglais *Epidermal Growth Factor*)*,* les renouvellements 242 de milieu ultérieurs, peuvent être effectués avec un milieu de culture 3 comprenant de l'acide ascorbique et de l'EGF. Selon un exemple, le premier renouvellement 242 de milieux à lieu 24 à 48h après l'ensemencement 23 des fibroblastes 1100.

Notons que selon le type des autres types cellulaires susceptibles d'être ensemencés avec les fibroblastes 1100, le milieu peut être configuré pour s'adapter et mimer l'environnement plus favorable aux types cellulaires ensemencés.

Le procédé 2 peut comprendre en outre un deuxième ensemencement 26, par exemple effectué après une ou plusieurs semaines de croissance cellulaire 24 des fibroblastes 1100. Préalablement au deuxième ensemencement 26, la matrice poreuse 10 peut être séchée 25 de façon similaire au séchage 22 décrit précédemment. Ensuite, un deuxième type 111 de cellules biologiques 11 peut être ensemencé 26, comprenant notamment des kératinocytes 1110.

Les séchages 22, 25 préalables aux ensemencements permettent de favoriser l'adhésion cellulaire des cellules 11 ensemencées sur la matrice poreuse, et donc leur croissance cellulaire subséquente, comme indiqué précédemment pour le séchage 22. En outre, le milieu de culture peut être renouvelé ou changé. Le séchage de la matrice poreuse 10 peut comprendre une aspiration de la solution ou du milieu cellulaire présent dans la matrice poreuse 10. Selon un exemple, lors du séchage, la matrice poreuse 10 peut en outre être déposée sur un support absorbant. La matrice poreuse 10 peut par exemple être déposée sur une compresse stérile, de préférence déposée sur un papier buvard stérile. De préférence, la matrice poreuse 10 est déposée sur le support absorbant après l'aspiration. L'aspiration permet de retirer la solution ou le milieu présent dans la matrice poreuse 10 de façon contrôlée et précise. Le dépôt sur le support absorbant permet de finaliser le séchage de la matrice poreuse 10 et ainsi d'améliorer encore l'adhésion cellulaire des cellules 11 ensemencées sur la matrice poreuse 10.

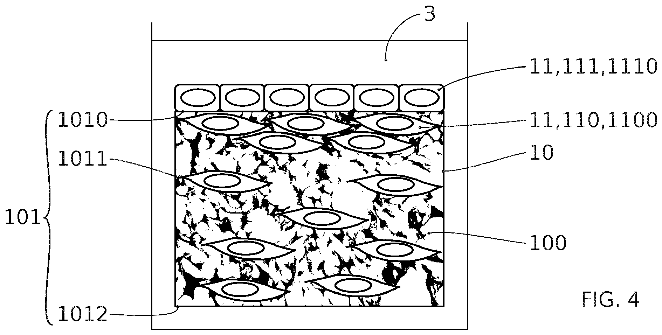

Les kératinocytes 1110 peuvent être ensemencés 26 par le dépôt 260 d'une suspension cellulaire, par exemple en goutte à goutte sur la surface 101 de la matrice poreuse 10, et notamment sur sa face supérieure 1010 selon l'exemple illustré en figure 4. De préférence, on évite le dépôt de la suspension cellulaire sur les faces latérales 1011 de la matrice poreuse 10 pour éviter la propagation des kératinocytes 1110 sur ces faces. Les kératinocytes 1110 peuvent être ensemencés à une densité cellulaire comprise entre 500 000 et 1 000 000 cellules/cm², voire sensiblement 800 000 cellules/cm². Notons à nouveau que selon le type de cellules biologiques ensemencées, la densité cellulaire peut être adaptée. Les kératinocytes 1110 ensemencés 26 restent à la surface extérieure 101 de la matrice poreuse 10 de par grâce à la présence de matrice extra cellulaire sécrétée par les fibroblastes et par la présence des fibroblastes eux-mêmes. Pour faciliter encore l'adhésion des kératinocytes 1110 ensemencées 26, le procédé peut en outre comprendre une étape d'adhésion des cellules 11, durant laquelle la matrice poreuse 10 est laissée pendant un temps déterminé, par exemple plusieurs minutes, voire une heure, et de préférence dans une atmosphère contenant 5 % en CO₂ et à 37°C.

Le procédé 2 comprend ensuite une croissance cellulaire 27 des kératinocytes 1110 ensemencés 26. Ainsi, les kératinocytes 1110 se multiplient par division cellulaire à la surface extérieure 101 de la matrice poreuse 10 jusqu'à la confluence 2702 des kératinocytes 1110, comme illustré par la figure 4.

Les kératinocytes 1110 en surface 101 de la matrice poreuse 10, forment ainsi un substitut épidermique équivalent à l'épiderme natif. L'on comprend qu'avec les fibroblastes 1100, voire avec leur matrice extracellulaires 112, le biomatériau 1 peut former un substitut dermo-épidermique équivalent à la peau native.

Pour cela, la croissance cellulaire 27 des kératinocytes 1110 peut comprendre une première sous-croissance 270 durant laquelle la matrice poreuse 10 avec les cellules ensemencées 23, 26 est immergée dans un milieu de culture 3 configuré pour mimer l'environnement natif des kératinocytes 1110, par exemple un milieu appelé MC2, adapté du milieu « Green »tel que décrit ultérieurement. L'ajout du milieu de culture 3 est de préférence réalisé sans que le flux de milieu 3 incident ne touche directement la surface 101 de la matrice poreuse 10, de façon à ne pas perturber les kératinocytes 1110 adhérés, et jusqu'à recouvrir la matrice poreuse et les cellules, y compris les kératinocytes 1110. La matrice 10 étant poreuse, le milieu de culture 3 est ainsi réparti de façon homogène dans la matrice poreuse 10 pour faciliter la croissance cellulaire 27. En outre, durant la croissance cellulaire 26, le milieu de culture 3 peut en outre être renouvelé 2701, typiquement à une fréquence de plusieurs fois par semaine.

Suite à une première immersion dans un milieu de culture 3 exempt acide ascorbique et d'EGF, les renouvellements 2701 de milieu ultérieurs, peuvent être effectués avec un milieu de culture 3 comprenant de l'acide ascorbique et de l'EGF. Selon un exemple, le premier renouvellement 2701 de milieux a lieu 24 à 48h après l'ensemencement 26 des kératinocytes 1110.

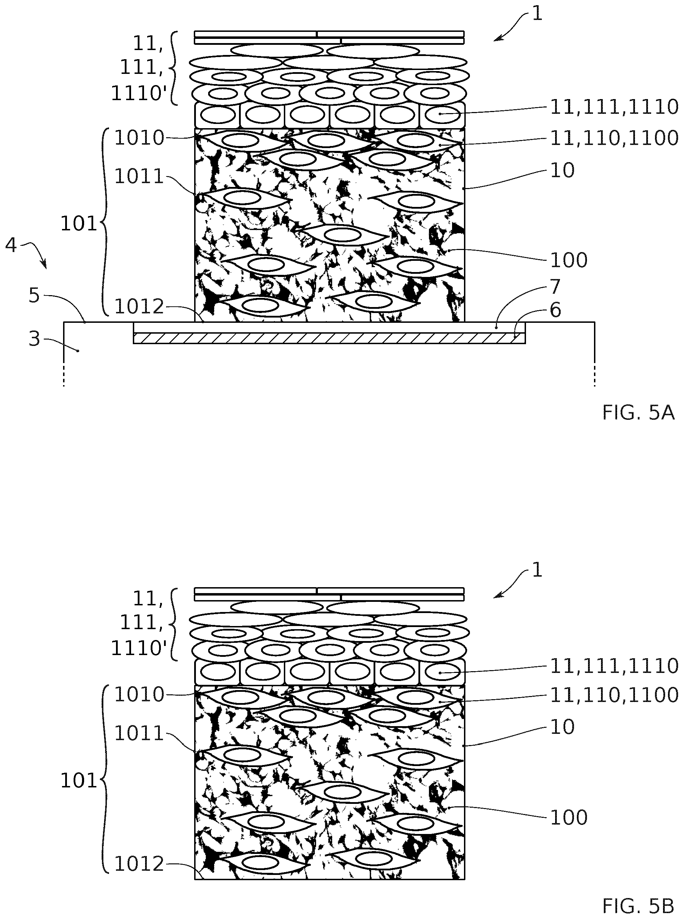

Suite à la première sous-croissance, la croissance cellulaire 27 des kératinocytes 1110 peut comprendre une maturation 271 durant laquelle la matrice poreuse 10 est placée à une interface 5 entre de l'air 4 et un milieu de culture 3. Cette configuration permet d'induire une différenciation 2710 des kératinocytes 1110' pour former un épiderme complexe, voire complet, caractérisé par une pluri-stratification et une différenciation comme illustré en figure 5A. En outre, lors et suite à la maturation, il est possible d'observer l'expression de protéines ou lipides spécifiques liées à la différenciation des kératinocytes 1110, telles que la cytokératine 1 ou 10, la loricine, l'involucrine ou la filaggrine, ou à leur état prolifératif, telles que la cytokératine 5 ou 14, ki67. Le biomatériau 1 se rapproche encore d'une peau native, notamment en termes de diversité des cellules biologiques présentes dans le tissu, et de fonctions physiologiques. Plus particulièrement, les kératinocytes 1110' peuvent se différencier pour former au moins une partie, voire l'ensemble des couches de l'épiderme suivantes, données de l'intérieur vers l'extérieur :
- *stratum germinativum :* composée d'une assise unique de kératinocytes à la jonction avec le derme, ces cellules étant capables de proliférer,
- *stratum spinosum :* composée de 5 à 6 assises de kératinocytes polygonaux,
- *stratum granulosum :* composée de 2 à 4 couches de kératinocytes aplatis et fusiformes,
- *stratum corneum.*

Selon l'exemple illustré en figure 5A, la matrice poreuse 10 avec les cellules ensemencées 23, 26 peut être disposée sur un support absorbant 7, placé sur une grille 6, par exemple en métal inoxydable et stérilisable par autoclave. Un milieu de culture 3 adapté, désigné milieu Air/liquide, peut ensuite être ajouté de façon à affleurer le support absorbant 7, par exemple un papier buvard. Ainsi, la matrice poreuse est maintenue hydratée tout en permettant l'induction de la différenciation des kératinocytes 1110. De préférence, le niveau du milieu de culture 3 est ajusté de façon à éviter la présence de bulle sous la grille 6, voire entre le support absorbant 7 et la grille 6. En outre, durant la maturation 271, le milieu de culture Air/liquide, de préférence additionné uniquement de vitamine C, peut en outre être renouvelé 2711, typiquement à une fréquence de plusieurs fois par semaine.

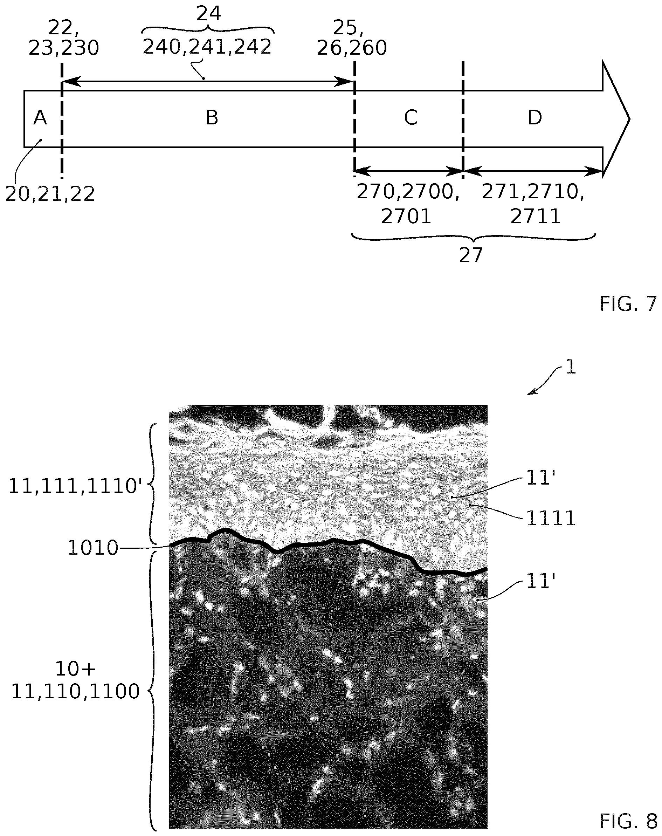

À titre d'exemple, une chronologie des étapes du procédé 2 de fabrication est donnée en référence à la figure 7. Dans une première phase A, la matrice poreuse 10 est fournie 20, puis préparée par hydratation 21 et séchage 22. Les fibroblastes 1100 peuvent ensuite être ensemencés 23 à un jour désigné J0. Dans une deuxième phase B, la croissance cellulaire 24 des fibroblastes peut être réalisée, suite à quoi les kératinocytes 1110 peuvent être ensemencés 26, 14 jours à 21 jours après J0. Dans une troisième phase C, la première sous-croissance 270 des kératinocytes 1110 peut être réalisée. 21 à 28 jours après J0, la matrice poreuse 10 peut être disposée à l'interface 5 entre l'air et le milieu de culture 5 pour permettre la différenciation cellulaire 2710 dans une phase D, par exemple durant 7 à 21 jours. Notons que les durées indiquées peuvent varier selon les besoins et la quantité de cellules disponible.

De par les caractéristiques du procédé 2 décrites, on comprend que l'on peut obtenir le biomatériau 1 selon le premier aspect de l'invention, comprenant notamment au moins un type 110 de cellules biologiques 11 vivantes d'un tissu, disposé dans le volume intérieur 100. En alternative ou en complément, le type 110 de cellules biologiques 11 vivantes d'un tissu peuvent être disposées sur la surface 101 de la matrice poreuse 10. Notamment, la disposition des cellules 11 dans la matrice poreuse 10 peut dépendre de la nature de cellules biologiques11 ensemencées Dans le cas de la cornée, des cellules stromales de la cornée peuvent être déposées dans la matrice poreuse 10 pour former le stroma, et des kératocytes peuvent être déposées à la surface de la matrice poreuse 10 pour former l'épithélium de la cornée. Plus particulièrement, le biomatériau 1 peut former un substitut dermo-épidermique d'une peau native, comme illustré en figure 5B.

Le biomatériau 1 peut notamment être utilisé pour une application *in vitro* de substances pharmaceutiques ou de substances cosmétiques. Lors du développement de l'invention, il a été mis en évidence que le procédé décrit permet d'obtenir le matériau 1 de façon reproductible. ailleurs, le biomatériau 1 peut être utilisé comme tissu modèle, par exemple pour la recherche fondamentale et dermatologique.

De par les caractéristiques précédemment décrites, le biomatériau 1 présente une activité régénératrice. Le biomatériau 1 est ainsi particulièrement adapté pour le traitement de lésions et/ou pour une greffe, par exemple sur la peau, sur l'oeil ou sur un os.

Le biomatériau 1 pouvant former un substitut dermo-épidermique d'une peau native, obtenu de façon reproductible, le biomatériaux 1 est particulièrement adapté pour le test de nouveaux produits dans l'industrie pharmaceutique. Par exemple, les peaux reconstruites à partir des cellules de patient peuvent représenter un modèle alternatif pour la modélisation de maladies dermatologiques, telles que le vitiligo, le psoriasis, la dermatite atopique et le *Xeroderma Pigmentosum,* et pour le développement des molécules thérapeutiques.

Le biomatériau 1 formant un substitut dermo-épidermique d'une peau native, le biomatériau 1 peut notamment être utilisé pour:
- traiter des séquelles de brûlures,
- traiter une plaie cutanée aigue
- traiter une plaie cutanée chronique
- traiter des zones donneuses de peau une fois un prélèvement tissulaire effectué,
- réaliser une greffe cutanée, par exemple suite à une brûlure, un ulcère, un traumatisme de la peau
- traiter toute perte de substance dermique ou dermo-épidermique.

Le biomatériau 1 formant un substitut dermo-épidermique d'une peau native, est particulièrement adapté pour le traitement des plaies chroniques ou d'une brûlure car il minimise, voire évite, l'ajout d'un traumatisme d'origine iatrogène, lié au prélèvement de peau au niveau d'une zone donneuse. En outre, les cellules biologiques 11 pouvant être autologues, le biomatériau 1 minimise le risque de rejet immunitaire du biomatériau 1.

Pour obtenir les cellules autologues 11, une biopsie de peau peut être réalisée, sur une surface saine et réduite par rapport à la surface nécessaire pour le traitement d'une plaie ou d'une brûlure. Les cellules 11 peuvent être multipliées par prolifération cellulaire dans le procédé de fabrication 2 afin d'obtenir une quantité suffisante pour permettre les croissances cellulaires 24 et/ou 27. Il est donc possible d'obtenir un biomatériau 1 présentant une surface adaptée à la surface de la zone lésée. Ainsi, le biomatériau 1 et son procédé de fabrication 2 permettent de minimiser les risques et traumatisme lié au prélèvement de peau chez une personne, par rapport aux solutions existantes.

En outre, le biomatériau 1 peut être utilisé comme substitut cutané pour le test *in vitro* de substances pharmaceutiques, par exemple de médicaments dermatologiques, et de produits dermo-cosmétiques. Les tests de l'efficacité et de l'innocuité des médicaments dermatologiques et des produits dermo-cométiques peuvent ainsi être effectués sur un biomatériau se rapprochant d'une peau native.

Des caractéristiques du biomatériau 1 ont été décrites lors de la description du procédé 2. Dans la suite, on détaille des caractéristiques additionnelles du biomatériau 1.

La matrice poreuse 10 du biomatériau 1 est maintenant décrite en détail. La matrice poreuse 10 peut être déformable élastiquement, par exemple sous la pression d'un doigt d'un utilisateur. Ainsi, l'utilisation du biomatériau 1 est facilitée, par exemple pour son application sur une zone lésée. Les propriétés de barrière du biomatériau 1 peuvent par exemple être améliorées.

La matrice poreuse 20 est résorbable. La matrice poreuse 20 peut en outre être biocompatible, notamment en étant constituée de matériaux biocompatibles. La matrice poreuse peut plus particulièrement être formée d'un matériau comprenant du collagène. Le collagène peut être du collagène bovin ou porcin de type I. Le matériau de la matrice poreuse 10 peut comprendre au moins 90%, de préférence au moins 95 %, et plus préférentiellement au moins 99 % en masse de collagène, par rapport à la masse sèche de la matrice poreuse 10. Ainsi, l'intégration dans l'organisme d'une partie du biomatériau 1, et plus particulièrement du tissu formé par les cellules biologiques 11 est facilitée.

En outre, le matériau de la matrice poreuse 10 peut être exempt de chitosan. Le chitosan présente une vitesse de dégradation dans un organisme inférieure à celle du collagène. Comme le biomatériau 1 comprend des cellules biologiques 11, il n'est pas nécessaire d'attendre que les cellules d'une personne colonisent le biomatériau suite à son implantation, par exemple par greffe, comme pour les solutions existantes. Dès lors, il est possible d'utiliser une matrice poreuse à base d'un matériau présentant une vitesse de dégradation plus rapide. Il peut être même avantageux d'accélérer la dégradation de la matrice poreuse pour accélérer l'intégration du tissu formé par les cellules biologiques.

La matrice poreuse 10 est sous la forme d'une éponge. Ainsi, la matrice poreuse peut être mise en forme en deux, voire en trois dimensions selon les besoins. La matrice poreuse 10 est une éponge hémostatique. Une éponge hémostatique peut aisément être mise en forme par découpe et présente une dureté lui permettant de conserver la forme voulue, tout en restant suffisamment déformable pour pourvoir être appliquée sur un support, par exemple sur le corps d'une personne. Une éponge hémostatique est une éponge synthétique généralement composée de matériaux d'origine biologique, et résorbable par l'organisme. La porosité de ces éponges permet une grande absorption de fluide arrivant, typiquement jusqu'à 35 fois leur poids. Une éponge hémostatique se présente comme un matériau sec, souple, poreux et disponible commercialement sous diverses formes et marques commerciales. Ainsi, la matrice poreuse 10 peut être disponible commercialement et à un coût réduit.

Une éponge hémostatique permet d'absorber le sang issu du corps humain et animal, par exemple issu d'une plaie sur laquelle est déposé le biomatériau. Une éponge hémostatique peut absorber un poids en sang sensiblement supérieur ou égal à 10 fois, de préférence sensiblement supérieur ou égal à 20 fois, et plus préférentiellement sensiblement supérieur ou égal à 30 fois le poids de l'éponge avant absorption. L'éponge hémostatique produit ainsi une pression sur le site du saignement, induisant une agrégation plaquettaire et donc activant les voies de la coagulation de la fibrine pour atteindre une hémostase. On pourrait toutefois s'attendre à ce que le sang aspiré dégrade les cellules du biomatériau. Or, lors du développement de l'invention, il a été constaté de façon surprenante que l'aspiration du sang dans le biomatériau 1 ne nuisait pas aux cellules 11 ensemencées dans le biomatériau 1, notamment lorsque le biomatériau 1 comprend une matrice extracellulaire 112 sécrétées par les cellules 11.

La matrice poreuse 10 fournie 20 est de préférence stérile, afin d'éviter la prolifération d'organismes non désirés dans le biomatériau 1. Selon un exemple, la matrice poreuse 10 est un produit suivant les Bonnes Pratiques de Fabrication (abrégé BPF, et pouvant être traduit en anglais par *Good Manufacturing Practices,* abrégé GMP). Les bonnes pratiques de fabrication sont des textes réglementaires établis par des États, la Commission européenne ou l'Organisation mondiale de la santé, et s'appliquent notamment à la fabrication de médicaments à usage humain ou vétérinaire. De façon équivalente, la matrice poreuse 10 est utilisable cliniquement sur un animal ou une personne, par exemple pour son application sur une plaie. Ainsi, le processus de mise sur le marché du biomatériau est simplifié.

Par exemple, la matrice poreuse 10 est une éponge hémostatique BPF choisie parmi les éponges hémostatiques des marques suivantes : Spongostan^{™}, CuraSpon^{®}, Gelfoam^{®} et Surgifoam^{®}.

À titre d'exemple, des caractérisations immunohistologiques par microscopie à fluorescence du biomatériau 1 (non revendiqué) sont illustrées par les figures 8 à 10. Pour ces caractérisations, des anticorps spécifiques à certaines structures cellulaires, et couplé à une sonde fluorescente sont appliquées sur le biomatériau 1.

La figure 8 représente une image de microscopie à fluorescence en coupe transversale du biomatériau, dans laquelle les noyaux 11' des cellules biologiques 11 et la cytokératine 14 1111 sont marqués. On peut observer la disposition des fibroblastes dans la matrice poreuse 10 et celles des kératinocytes au-dessus de la face supérieure 1010 de la matrice poreuse 10. Les kératinocytes différenciés 1110' présentent en outre un cytosquelette de cytokératine 1111.

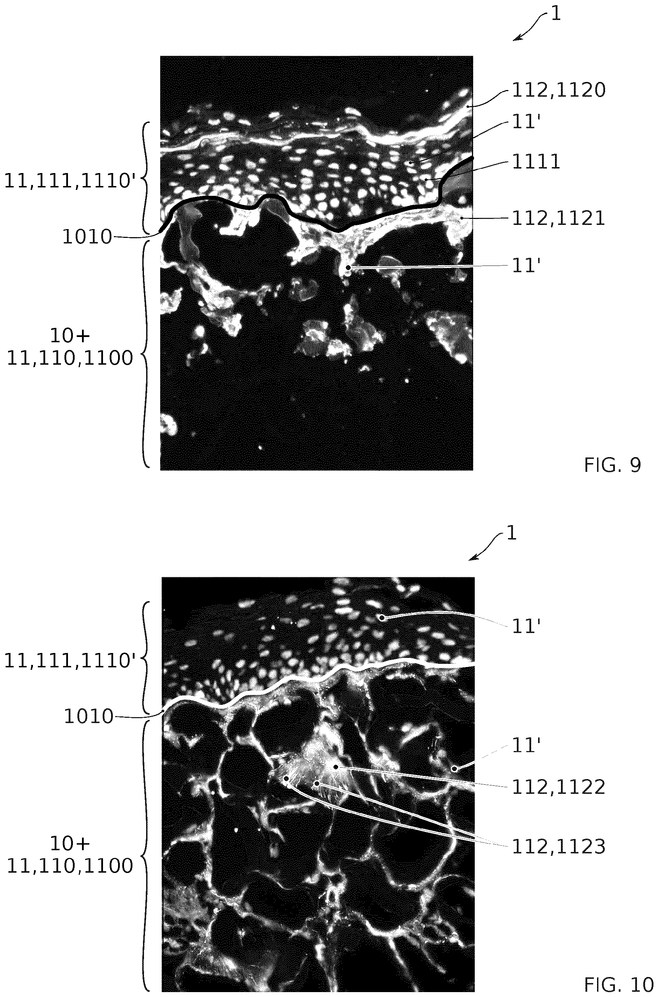

La figure 9 représente une image de microscopie à fluorescence en coupe transversale du biomatériau 1, dans laquelle les noyaux 11' des cellules biologiques 11, la filaggrine 1120 et le collagène de type I 1121 sont marqués. On peut observer la matrice extracellulaire 112 sécrétée, par le marquage des protéines énoncées, respectivement en dessous et en dessus de la face supérieure 1010 de la matrice poreuse 10.

La figure 10 représente une image de microscopie à fluorescence en coupe transversale du biomatériau 1, dans laquelle les noyaux 11 des cellules biologiques, la fibrilline 1 1122 et l'élastine 1123 sont marqués. On peut observer la matrice extracellulaire 112 sécrétée par les fibroblastes 1100 dans la matrice poreuse 10, par le marquage des protéines énoncées.

### Exemple de mode opératoire du procédé de fabrication du biomatériau

À titre d'exemple, un mode opératoire du procédé 2 de fabrication du biomatériau 1 est maintenant décrit selon un mode particulier de réalisation.

La composition des milieux de culture 3 utilisés est donnée dans les tableaux suivants. L'abréviation « qsp » signifie quantité suffisante pour le volume de solution voulue. Les pourcentages sont donnés en volume par rapport au volume de solution voulue.

### Milieu de culture MC1

**Tableau 1**

| Composants | Concentrations |
|---|---|
| Milieu DMEM/glutamax | qsp |
| Sérum de veau | 5% |
| Sérum de veau fœtal | 5% |
| EGF | 10 ng/mL |
| Pénicilline | 100 unité/mL |
| Streptomycine | 100 µg/mL |
| Amphotéricine B | 1 µg/mL |
| Vitamine C | 82, 2µg/mL |

### Milieu culture MC2 adapté du Milieu GREEN

**Tableau 2**

| Composants | Concentrations | Alternatives pour la clinique |
|---|---|---|
| Milieu DMEM/glutamax | qsp | |
| HAM F12 | qsp | |
| Sérum de veau fœtal | 10 % | |
| Hydrocortisone | 0,4 µg/mL | |
| Insuline | 5 µg/mL | Umuline^{®} à 0,12 UI/ml |
| Choleratoxine | 10⁻¹⁰ mol/L | Isuprel^{®} à 0,4µg/ml |
| Adénine | 24,3 µg/mL | |
| Tri lodo Thyronine | 2 nM | |
| EGF | 10 ng/ml | |
| Amphotéricine B | 100 unité/mL | |
| Pénicilline | 100 µg/mL | |
| Streptomycine | 1 µg/mL | |
| Vitamine C | 82,2 µg/mL | |

Notons qu'en vue d'une utilisation clinique, le milieu MC2 peut être adapté par l'homme du métier pour être compatible avec une utilisation clinique.

### Milieu de culture A/L

**Tableau 3**

| Composants | Concentrations |
|---|---|
| Milieu DMEM/glutamax | qsp |
| HAM F12 | qsp |
| Albumine de sérum bovin (BSA) | 8 mg/mL |
| Hydrocortisone | 0,4 µg/mL |
| Insuline | 5 µg/mL |
| Amphotéricine B | 1 µg/mL |
| Pénicilline | 100 unité/mL |
| Streptomycine | 100 µg/mL |
| Vitamine C | 82,2 µg/mL |

La préparation des réactifs pour fabriquer les milieux de culture est maintenant décrite :
- préparation de la trio-iodo-L-thyronine (abrégé T3 dans la suite):
   ∘ préparation d'une solution A à 2×10⁻⁴ M en pesant 13,6mg de T3, puis les dissoudre dans 1,5ml d'une solution d'hydroxyde de sodium NaOH à 0,02 mol/L, compléter à 100 mL avec de l'eau stérile,
   ∘ préparation de la solution mère de 2×10⁻⁶ M en réalisant une dilution au 1/100^{ème} de la solution A,
   ∘ filtrer sur un filtre de dimension 0,22 µm,
   ∘ aliquoter en portions de 550µL et congeler à -20°C,
   ∘ utiliser 500µl de la solution à 2×10⁻⁶M pour 500 mL de milieu MC2 pour obtenir une concentration finale 2×10⁻⁹ M,
- préparation de l'adénine :
   ∘ dissoudre 0,486g d'adénine dans 3mL d'une solution de NaOH à 0,4 mol/L. Ajouter 10 mL d'eau stérile puis 10 mL d'une solution d'acide chlorhydrique HCl à 1 mol/L. Mélanger et compléter avec de l'eau stérile jusqu'à 200 mL,
   ∘ filtrer sur un filtre de porosité 0,22µm,
   ∘ aliquoter en portions de 5 mL et congeler à -20°C
   ∘ utiliser 5 mL pour 500 m L de milieu pour obtenir une concentration finale à 24,3 µg/mL,
- préparation de l'EGF :
   ∘ préparer une solution à 10 µg/mL en reprenant 200 µg d'EGF lyophilisé par de l'eau stérile en qsp 20 mL,
   ∘ filtrer sur un filtre de porosité 0,22 µm,
   ∘ aliquoter en portions de 500µL et congeler à -20 °C,
   ∘ utiliser 500 µL pour 500 mL de milieu au moment de l'utilisation du milieu de culture, pour obtenir une concentration finale de 10 ng/mL,
- Préparation de la vitamine C :
   ∘ peser 2,5g de vitamine C en poudre et les dissoudre progressivement dans 60,8 mL de milieu DMEM à 37°C pour obtenir une concentration de 41,1 mg/mL ou de façon équivalente 142 mmol/L,
   ∘ filtrer sur un filtre de porosité 0,22 µm,
   ∘ aliquoter en portions de 0,5ml et congeler à -20°C
   ∘ Utilliser 200 µL dans 100mL de milieu pour obtenir une concentration finale de 82,2 µg/mL,
- préparation de la BSA :
   ∘ peser 4 g de BSA et les dissoudre dans 20 mL de milieu DMEM à 37°C,
   ∘ filtrer sur un filtre de porosité 0,22 µm,
   ∘ utiliser les 20 mL pour 500 mL de milieu,
- préparation de l'hydrocortisone :
   ∘ préparer une solution B à 5 mg/mL en diluant 25 mg dans 5 mL d'éthanol à 95%,
   o préparer une solution C à 200 µg/mL en prendre 0,4 mL de la solution B dans 9,6 mL (qsp 10mL),
   o aliquoter la solution C en portions de 1mL,
   o utiliser 1mL pour 500 mL de milieu, pour obtenir une concentration finale de 0,4 µg/mL,
- préparation de l'insuline :
   ∘ re-suspendre 50 mg d'insuline avec 500 µL d'une solution d'HCL à 0,1 mol/L,
   o diluer la suspension obtenue dans de l'eau stérile qsp 10 mL, pour obtenir une concentration de 5 mg/mL,
   o filtrer sur un filtre de porosité 0,22 µm,
   o aliquoter en portions de 500 µL,
   o utiliser 500µL pour 500mL de milieu, pour obtenir une concentration finale de 5 µg/mL,
- préparation de la choleratoxine :
   ∘ re-suspendre 1 mg de choleratoxine dans 12 mL d'eau stérile pour obtenir une concentration de 10.10⁻⁷ mol/L,
   o utiliser 500 µL de la solution à 10.10⁻⁷ pour 500 mL de milieu pour obtenir une concentration finale de 10⁻¹⁰ mol/L.

Les étapes du mode opératoire sont maintenant détaillées.

Pour l'extraction des fibroblastes et kératinocytes d'une biopsie cutanée d'un patient, la biopsie de peau est incubée durant 3 heures à 37°C dans une solution de dispase Il stérile. Le derme et l'épiderme sont alors séparés à l'aide de pinces stériles puis rincés dans du PBS 1X stérile.

Pour l'extraction des fibroblastes, les morceaux de derme obtenus sont ensuite mis dans une solution stérile de collagénase A à 0,5 mg/mL, sous agitation, pendant 4 à 6 heures à 37°C. Puis la solution est filtrée sur un tamis de porosité 70 µm et centrifugée à 1200 rotations par minutes (abrégé rpm dans la suite) durant 5 à 10 minutes. Après re-suspension du culot, la numération et la viabilité cellulaire est évaluée. Les fibroblastes sont ensemencés à une densité de 8 000 cellules/cm².

Pour l'extraction des kératinocytes, les morceaux d'épiderme sont incubés dans de la trypsine-EDTA 0,25% à 37°C pendant 15 minutes. Après ajout de solution inhibitrice de trypsine, la suspension de kératinocytes est ensuite filtrée sur tamis de porosité 70 µm, centrifugée à 1200 rpm durant 5 à 10 minutes et le culot de cellules ainsi obtenu est re-suspendu dans du milieu de culture pour évaluation de la numération et de la viabilité. Les kératinocytes sont ensemencés à la densité de 8000 cellules/cm².

Les fibroblastes et les kératinocytes sont cultivés à 37°C en atmosphère humide avec 5% de CO₂, les milieux sont renouvelés 3 fois par semaine et les cellules sont passées à sub-confluence par l'action de la trypsine EDTA.

Pour la préparation de la matrice poreuse, la matrice poreuse est hydratée durant 6 à 48 heures préalablement à l'ensemencement des cellules par un bain PBS 1X dont le volume est suffisant pour recouvrir la face supérieur de la matrice. Le PBS peut être ensuite aspiré. Un volume de milieu MC1, sans antibiotiques, ni EGF, ni vitamine C, le volume étant suffisant pour immerger en recouvrant la face supérieure de la matrice, est ajouté afin d'équilibrer le pH et l'osmolarité des matrices. En alternative à ce mode de préparation préférentiel, on peut prévoir d'hydrater la matrice poreuse par un bain de 2 à 3 heures en PBS 1X.

Pour l'ensemencement des fibroblastes, les fibroblastes sont décollés et dissociés par action enzymatique et dénombrés. Une suspension cellulaire pour obtenir une densité cellulaire de 0.5.10⁶ cellules/cellules/cm² est préparée. Après avoir aspiré le milieu de culture pour sécher la matrice poreuse, la suspension de fibroblastes est déposée gouttes à gouttes de façon homogène sur la face supérieure de la matrice poreuse. Les fibroblastes laissés à adhérer durant 1 heure à l'incubateur à 37°C et en atmosphère à 5% en CO₂.

Un volume suffisant pour recouvrir la face supérieure de la matrice poreuse de milieu MC1 avec antibiotiques mais sans EGF ni vitamine C est ajouté pour la croissance des fibroblastes. Le milieu est renouvelé 3 fois par semaine durant 2-3 semaines avec un milieu MC1 avec ajout d'antibiotiques, EGF et vitamine C.

Pour l'ensemencement des kératinocytes, les kératinocytes sont décollés par action enzymatique et dénombrés. Une suspension cellulaire à 0,5.10⁶ cellulescm²) est préparée. Après avoir aspiré le milieu de culture du substitut dermique formé, ce dernier peut être transféré sur une compresse stérile pour absorber le maximum d'humidité.

La suspension cellulaire de kératinocytes est déposée en goutte à goutte de façon homogène à la surface de la matrice en faisant attention que la suspension cellulaire ne déborde pas sur les faces latérales de la matrice poreuse. Les kératinocytes sont laissés à adhérer durant 1 heure à l'incubateur à 37°C et en atmosphère à 5% en CO₂.

Pour la première sous-croissance, un volume suffisant pour recouvrir la face supérieure de la matrice de milieu MC2 avec antibiotiques mais sans EGF ni vitamine C est ajouté sur la matrice très délicatement sans toucher les échantillons. Le milieu de culture est renouvelé 3 fois par semaine durant 1 semaine avec un milieu MC2 avec ajout d'antibiotiques, EGF et vitamine C.

Pour la maturation, la matrice poreuse est déposée après aspiration du milieu de culture et séchage sur une compresse stérile sur un papier buvard stérile lui-même posé sur une grille métallique. Le volume de milieu A/L supplémenté en antibiotiques et vitamine C est ajusté pour que celui-ci affleure le niveau du papier buvard en évitant la présence de bulle coincée sous la grille métallique. Le milieu est renouvelé 3 fois par semaine avec le milieu A/L contenant antibiotiques et vitamine C.

Au vu de la description qui précède, il apparaît clairement que l'invention propose un biomatériau se rapprochant davantage d'un tissu natif.

L'invention n'est pas limitée aux modes de réalisations précédemment décrits et s'étend à tous les modes de réalisation couverts par les revendications.

On peut notamment prévoir que les durées de croissance et/ou la composition des milieux soient adaptés pour d'autres types de cellules biologiques.

### REFERENCES

- 1: Biomatériau
- 10: Matrice poreuse
- 100: Volume intérieur
- 101: Surface
- 1010: Face supérieure
- 1011: Face latérale
- 1012: Face inférieure
- 11: Cellules biologiques
- 11': Noyaux
- 110: Premier type
- 1100: Fibroblastes
- 111: Deuxième type
- 1110: Kératinocyte
- 1110': Kératinocyte différenciés
- 1111: Cytokératine 14
- 112: Matrice extracellulaire
- 1120: Filaggrine
- 1121: Collagène de type I
- 1122: Fibrilline
- 1123: Élastine
- 2: Procédé
- 20: Fourniture d'une matrice poreuse
- 21: Hydratation de la matrice poreuse
- 22: Séchage de la matrice poreuse
- 23: Premier ensemencement
- 230: Dépôt d'une suspension de cellules biologique
- 24: Première croissance cellulaire
- 240: Immersion de la matrice poreuse dans un milieu de culture
- 241: Synthèse de matrice extracellulaire
- 242: Renouvellement du milieu de culture
- 25: Séchage de la matrice poreuse
- 26: Deuxième ensemencement
- 260: Dépôt d'une suspension de cellules biologique
- 27: Deuxième croissance cellulaire
- 270: Première sous-croissance
- 2700: Immersion de la matrice poreuse dans un milieu de culture
- 2701: Renouvellement du milieu de culture
- 2702: Confluence des cellules biologiques
- 271: Maturation
- 2710: Différenciation cellulaire
- 2711: Renouvellement du milieu de culture
- 3: Milieu de culture
- 4: Air
- 5: Interface air/milieu de culture
- 6: Grille
- 7: Support poreux

## Revendications

1. Procédé (2) de fabrication d'un biomatériau (1), comprenant :
• une fourniture (20) d'une matrice poreuse (10) résorbable formée d'un matériau comprenant du collagène, la matrice poreuse (10) étant une éponge hémostatique, et présentant un volume intérieur (100) et une surface extérieure (101), puis
• une hydratation (20) de la matrice poreuse (10) de façon à équilibrer le pH et l'osmolarité de la matrice poreuse (10),
• suite à l'hydratation (20) de la matrice poreuse (10), un séchage (22) de la matrice poreuse (10),
• suite au séchage (22) de la matrice poreuse (10), un ensemencement (23) de la matrice poreuse (10) par au moins un type (110) de cellules biologiques (11) vivantes d'un tissu, puis
• une croissance cellulaire (24) de l'au moins un type (110) de cellules biologiques (10),
pour former un biomatériau (1) comprenant :
• la matrice poreuse (10),
• l'au moins un type (110) de cellules biologiques (11) vivantes d'un tissu, disposées dans le volume intérieur (100) et en alternative ou en complément sur la surface (101) de la matrice poreuse (10).

2. Procédé (2) selon la revendication précédente, dans lequel l'ensemencement (23) de la matrice poreuse (10) est réalisé par dépôt (230) d'une suspension de l'au moins un type (110) de cellules biologiques (11) sur la matrice poreuse (10).

3. Procédé (2) selon l'une quelconque des revendications précédentes, comprenant en outre, après un premier ensemencement (23) par un premier type (110) de cellules biologiques (11), voire après le premier ensemencement (23) et une partie au moins d'une croissance cellulaire (24) du premier type (110) de cellules biologiques (11), un deuxième ensemencement (26) de la matrice poreuse (10) par au moins un deuxième type (111) de cellules biologiques (11), distinct du premier type (110) de cellules biologiques (11) ensemencées, puis une croissance cellulaire (27) de l'au moins un deuxième type (111) de cellule biologiques (11).

4. Procédé (2) selon la revendication précédente, comprenant en outre un séchage (25) de la matrice poreuse (10) préalablement au deuxième ensemencement (26) de la matrice poreuse (10), le deuxième ensemencement (26) étant réalisé par dépôt (260) d'une suspension de l'au moins un deuxième type (111) de cellules biologiques (11) sur la matrice poreuse (10).

5. Procédé (2) selon l'une quelconque des revendications précédentes, dans lequel les cellules biologiques (11) comprennent des cellules choisies parmi des cellules de tissu conjonctif et des cellules épithéliales.

6. Procédé (2) selon la revendication précédente, dans lequel, lorsque les cellules biologiques (10) sont des cellules de tissu conjonctif, la croissance cellulaire (24) comprend une immersion (240) de la matrice poreuse (10) dans un milieu de culture (3).

7. Procédé (2) selon l'une quelconque des deux revendications précédentes, dans lequel, lorsque les cellules biologiques (10) sont des cellules de tissu épithélial, la croissance cellulaire (27) comprend une première sous-croissance (270) pendant laquelle la matrice poreuse est immergée (270a) dans un milieu de culture (3), jusqu'à une confluence des cellules biologiques (10) en surface de la matrice poreuse (10).

8. Procédé (2) selon la revendication précédente, dans lequel la croissance (27) comprend, suite à la première sous-croissance (270), une maturation (271) durant laquelle la matrice poreuse (10) est placée à une interface entre de l'air (4) et un milieu de culture (3).

9. Utilisation d'un biomatériau (1) fabriqué par le procédé selon l'une quelconque des revendications précédentes, le biomatériau (1) comprenant :
• une matrice poreuse (10) résorbable formée d'un matériau comprenant du collagène, la matrice poreuse (10) étant une éponge hémostatique, et présentant un volume intérieur (100) et une surface (101), et
• au moins un type (110) de cellules biologiques (11) vivantes d'un tissu, disposées dans le volume intérieur (100) et/ou sur la surface (101) de la matrice poreuse (10),
pour une application *in vitro* d'au moins une substance choisie parmi une substance pharmaceutique et une substance cosmétique.

## Patentansprüche

1. Verfahren (2) zum Herstellen eines Biomaterials (1), das Folgendes umfasst:
• Bereitstellen (20) einer resorbierbaren porösen Matrix (10), die aus einem Kollagen enthaltenden Material gebildet ist, wobei die poröse Matrix (10) ein blutstillender Schwamm ist und ein Innenvolumen (100) und eine Außenoberfläche (101) aufweist, anschließend
• Hydratisieren (20) der porösen Matrix (10), um den pH-Wert und die Osmolarität der porösen Matrix (10) auszugleichen,
• nach dem Hydratisieren (20) der porösen Matrix (10), Trocknen (22) der porösen Matrix (10),
• nach dem Trocknen (22) der porösen Matrix (10), Beimpfen (23) der porösen Matrix (10) mit mindestens einer Art (110) lebender biologischer Zellen (11) aus einem Gewebe, anschließend
• ein Zellwachstum (24) von mindestens einer Art (110) biologischer Zellen (10),
um ein Biomaterial (1) zu bilden, das Folgendes umfasst:
• die poröse Matrix (10),
• die mindestens eine Art (110) lebender biologischer Zellen (11) aus einem Gewebe, die im Innenvolumen (100) oder alternativ oder zusätzlich auf der Oberfläche (101) der porösen Matrix (10) angeordnet sind.

2. Verfahren (2) nach dem vorhergehenden Anspruch, wobei das Beimpfen (23) der porösen Matrix (10) durch das Abscheiden (230) einer Suspension der mindestens einen Art (110) biologischer Zellen (11) auf der porösen Matrix (10) durchgeführt wird.

3. Verfahren (2) nach einem der vorhergehenden Ansprüche, das ferner nach einem ersten Beimpfen (23) mit einer ersten Art (110) biologischer Zellen (11) bzw. nach dem ersten Beimpfen (23) und einem Teil mindestens eines Zellwachstums (24) der ersten Art (110) biologischer Zellen (11) ein zweites Beimpfen (26) der porösen Matrix (10) mit mindestens einer zweiten Art (111) biologischer Zellen (11), die sich von der ersten Art (110) ausgesäter biologischer Zellen (11) unterscheidet, und anschließend ein Zellwachstum (27) der mindestens einen zweiten Art (111) biologischer Zellen (11) umfasst.

4. Verfahren (2) nach dem vorhergehenden Anspruch, das ferner ein Trocknen (25) der porösen Matrix (10) vor dem zweiten Beimpfen (26) der porösen Matrix (10) umfasst, wobei das zweite Beimpfen (26) durch das Abscheiden (260) einer Suspension der mindestens einen zweiten Art (111) biologischer Zellen (11) auf der porösen Matrix (10) durchgeführt wird.

5. Verfahren (2) nach einem der vorhergehenden Ansprüche, wobei die biologischen Zellen (11) Zellen umfassen, die aus Bindegewebszellen und Epithelzellen ausgewählt sind.

6. Verfahren (2) nach dem vorhergehenden Anspruch, wobei, wenn die biologischen Zellen (10) Bindegewebszellen sind, das Zellwachstum (24) ein Eintauchen (240) der porösen Matrix (10) in ein Kulturmedium (3) umfasst.

7. Verfahren (2) nach einem der beiden vorhergehenden Ansprüche, wobei, wenn die biologischen Zellen (10) Epithelgewebezellen sind, das Zellwachstum (27) ein erstes Teilwachstum (270) umfasst, bei dem die poröse Matrix bis zu einer Konfluenz der biologischen Zellen (10) auf der Oberfläche der porösen Matrix (10) in ein Kulturmedium (3) eingetaucht wird (270a).

8. Verfahren (2) nach dem vorhergehenden Anspruch, wobei das Wachstum (27) nach dem ersten Teilwachstum (270) eine Reifung (271) umfasst, bei der die poröse Matrix (10) an einer Grenzfläche zwischen Luft (4) und einem Kulturmedium (3) platziert wird.

9. Verwendung eines Biomaterials (1), das durch das Verfahren nach einem der vorhergehenden Ansprüche hergestellt wird, wobei das Biomaterial (1) Folgendes umfasst:
• eine resorbierbare poröse Matrix (10), die aus einem Kollagen enthaltenden Material gebildet ist, wobei die poröse Matrix (10) ein blutstillender Schwamm ist und ein Innenvolumen (100) und eine Außenoberfläche (101) aufweist, und
• mindestens eine Art (110) lebender biologischer Zellen (11) aus einem Gewebe, die im Innenvolumen (100) und/oder auf der Oberfläche (101) der porösen Matrix (10) angeordnet sind,
für eine *In-vitro*-Anwendung mindestens einer Substanz, die aus einer pharmazeutischen Substanz und einer kosmetischen Substanz ausgewählt ist.

## Claims

1. A method (2) for manufacturing a biomaterial (1), comprising:
• supply (20) of a resorbable porous matrix (10) formed of a material comprising collagen, the porous matrix (10) being a haemostatic sponge, and having an interior volume (100) and an exterior surface (101), then
• hydration (20) of the porous matrix (10) so as to balance the pH and osmolarity of the porous matrix (10),
• following hydration (20) of the porous matrix (10), drying (22) of the porous matrix (10),
• following drying (22) of the porous matrix (10), seeding (23) of the porous matrix (10) with at least one type (110) of living biological cells (11) of a tissue, then
• a cellular growth (24) of at least one type (110) of biological cells (10),
to form a biomaterial (1) comprising:
• the porous matrix (10),
• at least one type (110) of living biological cells (11) of a tissue, disposed in the interior volume (100) and alternatively or additionally on the surface (101) of the porous matrix (10).

2. The method (2) according to the preceding claim, wherein the seeding (23) of the porous matrix (10) is carried out by depositing (230) a suspension of at least one type (110) of biological cells (11) on the porous matrix (10).

3. The method (2) according to any one of the preceding claims, further comprising, after a first seeding (23) with a first type (110) of biological cells (11), or even after the first seeding (23) and at least part of a cellular growth (24) of the first type (110) of biological cells (11), a second seeding (26) of the porous matrix (10) with at least a second type (111) of biological cells (11), distinct from the first type (110) of seeded biological cells (11), then a cellular growth (27) of the at least one second type (111) of biological cells (11).

4. The method (2) according to the preceding claim, further comprising drying (25) the porous matrix (10) prior to the second seeding (26) of the porous matrix (10), the second seeding (26) being carried out by depositing (260) a suspension of at least one second type (111) of biological cells (11) on the porous matrix (10).

5. The method (2) according to any one of the preceding claims, wherein the biological cells (11) comprise cells selected from connective tissue cells and epithelial cells.

6. The method (2) according to the preceding claim, wherein, when the biological cells (10) are connective tissue cells, the cellular growth (24) comprises an immersion (240) of the porous matrix (10) in a culture medium (3).

7. The method (2) according to any one of the two preceding claims, wherein, when the biological cells (10) are epithelial tissue cells, the cellular growth (27) comprises a first undergrowth (270) during which the porous matrix is immersed (270a) in a culture medium (3), until a confluence of the biological cells (10) on the surface of the porous matrix (10).

8. The method (2) according to the preceding claim, wherein the growth (27) comprises, following the first undergrowth (270), a maturation (271) during which the porous matrix (10) is placed at an interface between air (4) and a culture medium (3).

9. A use of a biomaterial (1) manufactured by the method according to any one of the preceding claims, the biomaterial (1) comprising:
• a resorbable porous matrix (10) formed from a material comprising collagen, the porous matrix (10) being a haemostatic sponge, and having an interior volume (100) and a surface (101), and
• at least one type (110) of living biological cells (11) of a tissue, disposed in the interior volume (100) and/or on the surface (101) of the porous matrix (10),
for an in vitro application of at least one substance selected from a pharmaceutical substance and a cosmetic substance.
